# EUROPEAN PATENT APPLICATION

(11) **EP 2 096 179 A1**
(43) Date of publication of application: **02.09.2009**
(21) Application number: 07850628.4
(22) Date of filing: 14.12.2007
(51) Int. Cl.: C12Q 1/68, C12M 1/00, C12M 1/34, G01N 21/27, G01N 27/327, G01N 33/543, G01N 33/574, G01N 35/00, G01N 35/08, G01N 37/00, C12N 15/09

(54) **SEPARATION/PURIFICATION METHOD AND MICROFLUID CIRCUIT**

(30) Priority: 22.12.2006 JP 2006346208
(71) Applicant: Rohm Co., Ltd., Kyoto-shi, Kyoto 615-8585 (JP)
(72) Inventor: NIWA, Daisuke, Kyoto-shi, Kyoto 615-8585 (JP)
(74) Representative: Prüfer & Partner GbR European Patent Attorneys
(86) International application number: PCT/JP2007/074124
(87) International publication number: WO 2008/078579

(57) **Abstract**

A simple and accurate separation purification method for object biomolecules or bio-associated substance through a particulate aggregative reaction is provided. A microfluidic circuit utilizing a particulate aggregate as a detection marker in a nonfluorescence method such as an electrochemical method or surface plasmon resonance is provided. The separation purification method comprises the steps of forming a particulate aggregate by a reaction between particulates modified with a labeling substance specifically reacting with a label object substance and biomolecules or a bio-associated substance containing the label object substance and separation-purifying the particulate aggregate. The microfluidic circuit utilizes this separation purification method.

SEQUENCE LISTING

<110>  Rohm Kabushiki Kaisha

<120>  Separation/Purification Method and Microfluid Circuit

<130>  06-00597

<160>  8

<170>  PatentIn version 3.1

<210>  1
<211>  20
<212>  DNA
<213>  Artificial

<220>
<223>  Inventor: Niwa, Daisuke

<220>
<223>  a degenerate primer sequence

<400>  1
ccccggacga tattgaacaa
    20

<210>  2
<211>  19
<212>  DNA
<213>  Artificial

<220>
<223>  a degenerate primer sequence

<400>  2
ccagacggaa accgtagct
    19

<210>  3
<211>  34
<212>  DNA
<213>  Artificial

<220>
<223>  a degenerate primer sequence

<400>  3
gcggggagca gcctcggttc actgaagacc cagg
    34

<210>  4
<211>  30
<212>  DNA
<213>  Artificial

<220>
<223>  a degenerate primer sequence

<400>  4
cgcgtggccc ctgcgacagg ggccaggagg
    30

<210> 5
<211> 19
<212> DNA
<213> Artificial

<220>
<223> a degenerate primer sequence

<400> 5
tggcattctg ggagcttca
 19


<210> 6
<211> 15
<212> DNA
<213> Artificial

<220>
<223> a degenerate primer sequence

<400> 6
cggcccctgc accag
 15


<210> 7
<211> 34
<212> DNA
<213> Artificial

<220>
<223> a degenerate primer sequence

<400> 7
ggggggagca gcctcggttc actgaagacc cagg
 34


<210> 8
<211> 30
<212> DNA
<213> Artificial

<220>
<223> a degenerate primer sequence

<400> 8
cccgtggccc ctgcgacagg ggccaggagg
 30

## Description

### TECHNICAL FIELD

The present invention relates to a separation purification method, utilizing an aggregative reaction of particulates, capable of rapidly and simply detecting gene mutation or a tumor marker and a microfluidic circuit utilizing this separation purification method.

### BACKGROUND ART

Following the recent development in the biochemical and medical fields, a microfluidic circuit capable of accurate, rapid and simple gene diagnosis and immunodiagnosis is desired. At present, a DNA microarray is employed in gene analysis for specifying a mutated site or the like by fluorescent detection through specificity of hybridization. With this fluorescent detection, however, it is difficult to cope with ulterior advanced medical care and tailor-made medical care, due to factors such as destabilization of a signal resulting from discoloration of a labeling agent, reduction of sensitivity, a high cost for a reagent and difficulty in scale-down of an apparatus. In order to solve these problems, therefore, a novel fluorescence labeling substance as well as a novel detection system substituting for the fluorescent system have been developed, an electrochemical method, surface plasmon resonance and a method employing a semiconductor transistor or the like have been proposed, and development of a detection marker material is also attempted.

In the present circumstances of the microfluidic circuit, on the other hand, it is difficult to conduct all reactions in a chip particularly in the field of gene analysis or the like, and a method carrying out reactions up to a polymerase chain reaction outside a chip and performing introduction into the chip immediately before detection similarly to a DNA microarray is carried out in general. While construction of a total fluorescence system using blood as a sample has recently been attempted, an accurate separation purification process for collected liquid and an object substance before and after a reaction and an impurity and an interfering substance has not yet been attained in the chip developed in the aforementioned manner, and the problem of insufficient response sensitivity is not yet solved. In order to improve the performance of a microsensor system, three elements, i.e., a detection method, a detection marker and purification of an object substance must be complementarily improved. In particular, a substance corresponding to a novel detection system substituting for the fluorescent system and playing a role also in separation purification of a detection marker conceivably contributes to improvement in performance.

As currently examined separation purification systems for object substances, methods such as two-liquid laminar flow separation and magnetic particulate separation are proposed. While molecule sorting by means of a photoalteration gel passage or the like can be listed as an example in the two-liquid laminar flow separation, a feed pump and an external light source are generally required in the case of this system, accompanied by complication of the reaction system following introduction of gel. While magnetic particulate separation is generally frequently employed for a separation purification process (refer to Japanese Patent Laying-Open No. 2005-312353 (Patent Document 1)), stability of the magnetic particulates themselves is low, self aggregation easily takes place and the magnetic particulates are relatively wide in diameter, resulting in such problems that reaction efficiency is low and detection is hardly applicable except for fluorescence. However, utilization of particulates in separation and aggregation is preferable in view of simplicity and rapidness, and it is expected that a separation purification process in a microfluidic circuit can be implemented by further contrivances of the material for the particulates, dimensional design etc. in relation to the process.

In an application employing an aggregative reaction as a marker for biodetection dissimilarly to the application of the separation purification to the magnetic particulates, particles of gold having an average particle diameter of about 10-odd nanometers (the particles of gold having the average particle diameter of the nanometer level are hereinafter also referred to as "gold nanoparticles") are used. In the case of detection of gene mutation, for example, a method of observing the presence or absence of an aggregation system accompanied by hybridization of two types of gold nanoparticles modified with DNA complementary to detection object DNA and the detection object DNA by turbidity and colorimetry is proposed (refer to National Patent Publication Gazette No. 2000-516460 (Patent Document 2), National Patent Publication Gazette No. 2003-503699 (Patent Document 3) and "Inorganic Chemistry, 39, 2000, 2258-2272" (Non-Patent Document 1)). Further, a method of detecting the degree of aggregation according to the presence or absence of cutting with an restriction enzyme in a reaction system between gold nanoparticles modified with a labeling substance and biomolecules modified with a label object substance or the like is known (refer to Japanese Patent No. 3545158 (Patent Document 4)). According to this method, only the presence or absence of an aggregative reaction and the presence or absence of aggregation/separation are detected, and separation purification of the aggregate itself is not carried out.

The conventional method has such a problem that 2n types of gold nanoparticles modified with the DNA complementary to the detection object DNA must inefficiently be prepared in response to the type n of the detection object DNA when utilizing the gold nanoparticles modified with DNA, and is insufficient in universality. In observation of change in the degree of aggregation through the restriction enzyme, the restriction enzyme is high-priced, those other than the typical one are hard to obtain and a necessary enzyme may not be present every application, and hence this method is inferior in diversity.

In order to further improve the accuracy and the sensitivity, therefore, it is important to supply a separation purification method for this aggregate system into the reaction system. In relation to this gold nanoparticle aggregation system, however, attention has heretofore been focused only on the utilization of the detection marker, and no system such as matching with a preferable detection method or application to a separation purification process utilizable in a total process has been proposed. While detection by the aforementioned aggregative reaction is ordinary photodetection, further improvement of the sensitivity is expected through utilization of a detection system such as the electrochemical method, the surface plasmon resonance or the method employing the semiconductor transistor.

In consideration of the high death rate resulting from cancer, demands for early diagnosis of cancer and a simple and rapid sensing system are increased nowadays. While a gene diagnosis chip directed to a cancer gene or the like has heretofore been developed, there is no revolution in the basic detection system as compared with the conventional DNA microarray. Tumor marker diagnosis is also regarded as effective as cancer diagnosis other than gene diagnosis, examination is often conducted in a clinical scene. However, this method is nothing but conventional blood diagnosis based on a microtube and chromatography, and inferior in simplicity and rapidness.

In consideration of such a cancer market, a high-performance diagnosis chip employing a microfluidic circuit conceivably makes a remarkable contribution to this field. As yet, however, no idea of implementation of a microfluidic circuit for cancer diagnosis has been observed. If a tumor marker can be detected and specific function gene mutation can be diagnosed with the same chip, rapid and simple early diagnosis and treatment transcending the conventional methods can conceivably be proposed.
Patent Document 1: Japanese Patent Laying-Open No. 2005-312353
Patent Document 2: National Patent Publication Gazette No. 2000-516460
Patent Document 3: National Patent Publication Gazette No. 2003-503699
Patent Document 4: Japanese Patent No. 3545158
Non-Patent Document 1: Inorganic Chemistry, 39, 2000, 2258-2272

### DISCLOSURE OF THE INVENTION

### PROBLEMS TO BE SOLVED BY THE INVENTION

An object of the present invention is to provide a simple and accurate separation purification method for object biomolecules or bio-associated substance through a particulate aggregative reaction. Another object of the present invention is to provide a microfluidic circuit utilizing a particulate aggregate as a detection marker in a nonfluorescence method such as an electrochemical method or surface plasmon resonance.

### MEANS FOR SOLVING THE PROBLEMS

The separation purification method according to the present invention comprises the steps of forming a particulate aggregate by a reaction between particulates modified with a labeling substance specifically reacting with a label object substance and biomolecules or a bio-associated substance containing the label object substance and separation-purifying the particulate aggregate. The particulates are preferably particles of gold having an average particle diameter of 1 nm to 500 nm. The particulate aggregate is preferably centrifugally separation-purified. The label object substance preferably contains an amplified gene prepared by amplifying a wild type gene or a mutant gene contained in body fluid or a tissue segment, and the body fluid or the tissue segment preferably contains the label object substance.

The microfluidic circuit according to the present invention comprises a specific reaction section, a separation purification section and a detection section, and the specific reaction section forms a particulate aggregate by a specific reaction between particulates modified with a labeling substance specifically reacting with a label object substance and biomolecules or a bio-associated substance containing the label object substance. The separation purification section separation-purifies the formed particulate aggregate, and the detection section detects the separation-purified particulate aggregate by a nonfluorescence method. According to this microfluidic circuit, gene mutation including single nucleotide polymorphism can be detected, and a cancer tumor marker can be qualitatively or quantitatively detected. At least a part of the specific reaction section, the separation purification section and the detection section preferably includes a plurality of chambers. Fluid can be centrifugally fed between the specific reaction section, the separation purification section and the detection section. The detection section preferably has a detection surface for fixing the particulate aggregate, and the particulates preferably contain particles of gold and particles of noble metal other than gold. The detection section preferably has an electrode for an electrochemical reaction, and detection can be performed by surface plasmon resonance. When using this microfluidic circuit, one or both of a cancer-associated gene and a tumor marker can be qualitatively or quantitatively detected in the same chip.

### EFFECTS OF THE INVENTION

The biomolecules or the bio-associated substance to be detected can be easily separation-purified, and qualitative or quantitative detection can be accurately and rapidly performed. In particular, analysis of gene mutation including single nucleotide polymorphism and detection of a tumor marker can be easily performed in the same microfluidic circuit, whereby whole blood, serum or tissue fluid can be simply tested, to be of helpful to tailor-made medical care.

### BRIEF DESCRIPTION OF THE DRAWINGS

Fig. 1 is a diagram showing the protocol of a reaction in Example 1.
Fig. 2 illustrates cross sections of microfluidic circuits according to the present invention.
Fig. 3 shows the outline of specific steps in Example 1.
Fig. 4 illustrates plasmon absorption peaks before an aggregative reaction, after the aggregative reaction and after separation purification in Example 1.
Fig. 5 shows fluorescence intensity levels of samples after gene amplification in a case of employing primers in WT and a case of employing primers in MT in preliminary examination of Example 1.

### DESCRIPTION OF THE REFERENCE SIGNS

1 specific reaction section, 2 separation purification section, 3 detection section.

### BEST MODES FOR CARRYING OUT THE INVENTION

An embodiment and Examples of the present invention are now described with reference to the drawings. The dimensional relation between the thicknesses, the magnitudes, the widths etc. in the drawings is properly changed in order to clarify and simplify the illustrations, and does not correspond to the actual dimensional relation.

### (Separation Purification Method)

The separation purification method according to the present invention is a method combining particulates such as gold nanoparticles modified with a labeling substance and biomolecules or a bio-associated substance containing a label object substance with each other so that the label object substance and the labeling substance specifically react with each other and form a particulate aggregate, and thereafter separation-purifying the formed particulate aggregate. A reaction-specific aggregative reaction such as an antigen-antibody reaction takes place between the label object substance and the labeling substance, so that a measurement object substance can be rapidly and accurately qualitatively or quantitatively detected by an electrochemical method or surface plasmon resonance in a state of an aggregate after capturing the measurement object as the aggregate, separation-purifying the aggregate and removing impurities.

A wild type gene or a mutant gene contained in body fluid or a tissue segment of whole blood, plasma, serum, tissue, tissue fluid, crine, claw, skin, sputum, urine or feces can be reaction-specifically amplified by a gene amplification method such as a LAMP (Loop-Mediated Isothermal Amplification) method, and the label object substance in the present invention contains such a selectively amplified gene. The label object substance contained in the body fluid or the tissue segment is so amplified that the label object substance can be efficiently qualitatively or quantitatively analyzed.

In the present invention, the particulates are prepared from particulates modified with the labeling substance specifically reacting with the label object substance. The particulates are modified with a labeling substance such as an antibody, an antigen or streptavidin respectively, to cause an aggregative reaction with the label object substance such as an antigen, an antibody or a biotinized reaction-specifically amplified gene, for example. Therefore, the surfaces of the particulates are preferably previously modified with molecules reacting with the labeling substance. The molecules reacting with the labeling substance and modifying the surfaces of the particulates are desirably prepared from alkanethiol molecules or an ionic polymer such as polyerucin. Such molecules are preferably straight-chain molecules having at least one COOH-, NH₂-, CF₃-, CH₃- or CN- as a terminal functional group.

The particulates are preferably prepared from particles of gold in a point that the same are a chemically stable substance having high simplicial purity and causing a specific electrochemical response and plasmon resonance. In particular, aggregative purification of gold nanoparticles effectively plays an important role for capture and detection of biomolecules accompanied by a specific reaction such as gene analysis for single nucleotide polymorphism and sensing of a reaction substrate accompanied by an antigen-antibody reaction, for example. In a point that particulates prepared from noble metal so form a particle mixture that optical properties and electrochemical properties can be amplified, particles of gold and particles of noble metal other than gold are preferably mixed with each other. Platinum, rhodium or palladium is effective as the noble metal other than gold.

The average particle diameter of the gold nanoparticles is preferably at least 1 nm, more preferably at least 2 nm, in order to develop the optical properties and the electrochemical properties specific to the gold nanoparticles. On the other hand, the average particle diameter of the gold nanoparticles is preferably not more than 500 nm, more preferably not more than 50 nm, so that the aggregate can be smoothly formed and easily centrifuged. In this specification, the particle diameters are measured by observation with a transmission electron microscope. The average particle diameter is calculated by direct measurement from an image of the transmission electron microscope. Gold nanoparticles of 1 nm to 10 nm self-aggregate in an aqueous solution if the surfaces thereof are not modified with the molecules reacting with the labeling substance or the like, and hence those previously modified with molecules of alkanethiol or the like in an organic solvent such as toluene are preferably employed.

The labeling substance causing the selective aggregative reaction with the label object substance is preferably prepared from a substance causing an antigen-antibody reaction and a specific vital reaction. The selectivity of the aggregative reaction can be improved by employing a selectively amplifiable system such as polymerase chain reaction (PCR), an ICAN (Isothermal and Chimeric primer-initiated Amplification of Nucleic acids) method or the LAMP method as a gene amplification reaction as the specific vital reaction, for example. The labeling substance employed for the aggregative reaction is preferably prepared from an antigen, an antibody or a gene, and preferably fixed to the particulates such as gold nanoparticles. A reaction system capable of sandwich immunoassay is preferable in the antigen-antibody reaction, while the gene is preferably modified with target molecules capable of reacting with the gold nanoparticles on both terminals. In this antigen-antibody reaction or gene amplification reaction, further, conditions are preferably scrutinized so that reaction specificity is attained, and the selectivity of aggregation/separation can be more improved. The aggregative reaction is preferably performed in an aqueous solution, in the temperature range of 15°C to 55°C. The aggregative reaction denotes such a reaction that the gold nanoparticles form a cluster due to the reaction between the label object substance and the labeling substance.

### (Microfluidic Circuit)

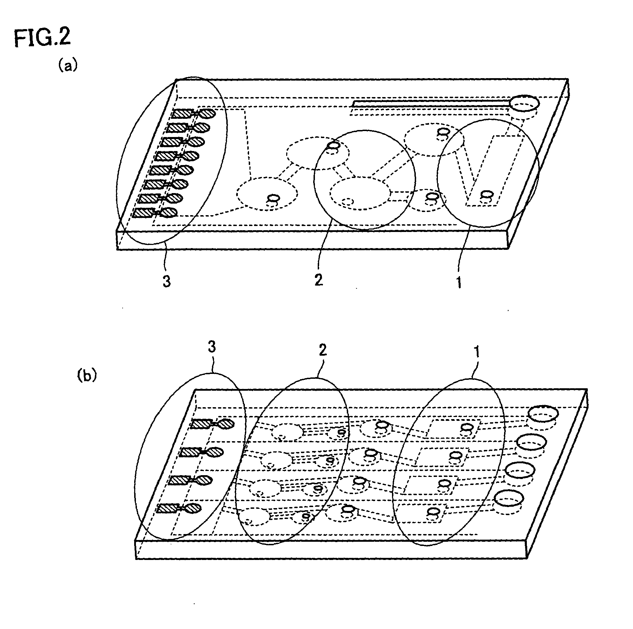

Figs. 2(a) and 2(b) illustrate cross sections of microfluidic circuits according to the present invention. The circuit shown in Fig. 2(a) or the circuit shown in Fig. 2(b) can be arbitrarily selected in response to the detection object or the like. As shown in each of Figs. 2(a) and 2(b), the microfluidic circuit comprises a specific reaction section 1, a separation purification section 2 and a detection section 3, and specific reaction section 1 forms a particulate aggregate by a specific reaction between particulates modified with a labeling substance specifically reacting with a label object substance and biomolecules or a bio-associated substance containing the label object substance. Then, separation purification section 2 separation-purifies the particulate aggregate, and detection section 3 detects the separation-purified particulate aggregate by a nonfluorescence method. The biomolecules or the bio-associated substance can be simply separation-purified through the aggregative reaction, and accurate detection can be implemented. Further, a test object can be easily detected by utilizing the particulate aggregate as a detection marker in the nonfluorescence method such as colorimetry, turbimetry, an electrochemical method or surface plasmon resonance. Therefore, cancer-derived gene mutation including single nucleotide polymorphism can be detected and a tumor marker can be qualitatively or quantitatively analyzed with one chip.

The particulate aggregate can be centrifugally separation-purified in the microfluidic circuit, the supernatant is discharged as waste liquid, and new liquid is introduced. The liquid can also be centrifugally fed between specific reaction section 1, separation purification section 2 and detection section 3. When a micropassage system in a liquid-liquid two-layer flow system is used, an aqueous solution containing biomolecules is introduced into a first inlet, another aqueous solution containing gold nanoparticles is introduced into a second inlet and the solutions are made to flow by external action, so that an aggregative reaction progresses on the liquid-liquid interface and the aggregate can be recovered in a passage closer to a first outlet.

When the separation-purified particulate aggregate is detected by the electrochemical method or surface plasmon resonance, the interfacial reaction can be sensitively captured, and measurement can be conducted without requiring a labeling agent. Detection section 3 is preferably provided with a detection surface for fixing the particulate aggregate, in order to simplify the detection and increase the detection accuracy. The particulate aggregate can be fixed by electrostatic adsorption, chemical bonding, physical adsorption or the electrochemical method. More specifically, the surface of an electrode in detection section 3 of the microfluidic circuit is so covered with an amino terminal monolayer that the particulate aggregate can be fixed to the surface of the electrode by interaction between biotin-streptavidin in the particulate aggregate and an amino group on the electrode, for example. The state of stability of the gold nanoparticles can be controlled by modification with functional molecules such as carboxyl groups or ferrocenyl groups, and electrochemical amplification can be performed in detection. When electrochemical fixation is performed, on the other hand, an ionic molecule-modified surface can be so formed by sweeping potential baser than redox potential for gold that the gold nanoparticles can be rapidly attracted to the substrate surface.

In detection section 3, gold, platinum, carbon or ITO can be employed as the electrode material for the electrochemical reaction when a redox labeling agent is employed. A material having a potential window not overlapping with the specific peak potential position of gold is desirable when no labeling agent is employed, and more specifically, carbon or ITO is preferable. In a surface plasmon resonance detection system, a material not overlapping with the adsorption peak of the gold nanoparticles is desirable, and silver or copper is preferable as metal while a substrate having a higher refractive index than an underlayer substrate is effective as base metal, for example. Quartz or ITO can be employed as a dielectric material, for example.

The presence or absence of specific object molecules can be determined from the degree of aggregation of the particulate aggregate such as a gold nanoparticle aggregate, for example. At least a part of specific reaction section 1, separation purification section 2 and detection section 3 preferably includes a plurality of chambers. The plurality of chambers are so provided that a large number of object substances can be detected by the same chip. A gene, an antibody and an antigen of a specific functional site subjected to gene amplification or the like can be listed as examples of the label object substance, and more specifically, one or both of a cancer-associated gene including single nucleotide polymorphism and a tumor marker can be qualitatively or quantitatively detected in the same chip.

While the present invention is now specifically described with reference to Examples, the present invention is not restricted to the following Examples.

### (Example 1)

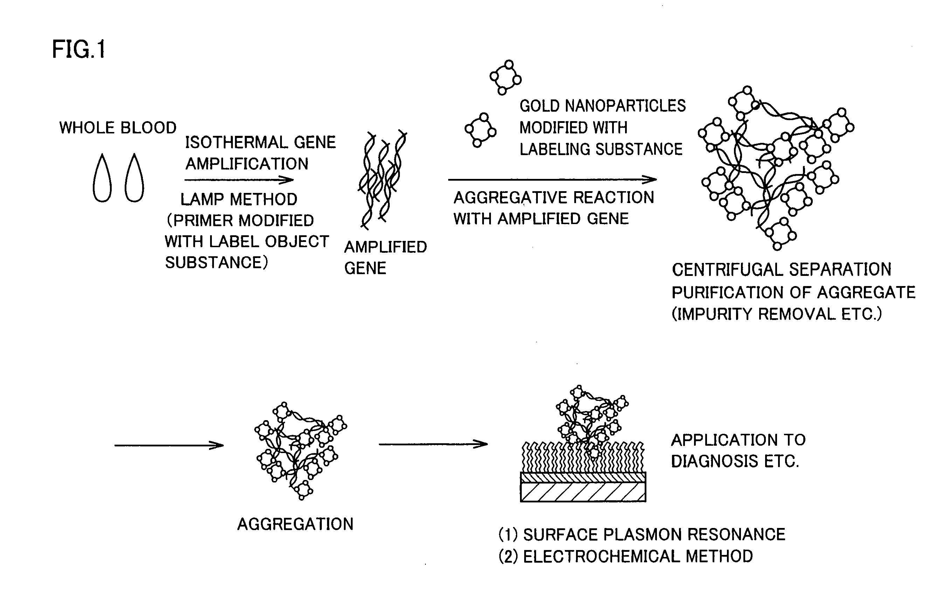

In this Example, a particulate aggregate was detected after performing separation purification of a cancer-derived amplified gene through a gold nanoparticle aggregative reaction. Fig. 1 shows the protocol of the reaction. As shown in Fig. 1, an amplified gene was formed by performing isothermal gene amplification on whole blood employed as a sample by the LAMP method with a primer modified with a label object substance, and a particulate aggregate was thereafter formed by reacting gold nanoparticles modified with a labeling substance and the amplified gene. Then, separation purification of the particulate aggregate was centrifugally performed for removing impurities, and the particulate aggregate was thereafter detected as such by surface plasmon resonance and the electrochemical method.

The gold nanoparticles were prepared from gold nanoparticles of 10 nm in average particle diameter modified with streptavidin which is the labeling substance specifically reacting with the label object substance, and an aqueous solution (by Sigma Aldrich, Japan) of the gold nanoparticles was employed in this Example. In order to detect mutation of the p53 gene extracted from the blood, the exon-4 gene in the p53 gene was amplified in this Example, for detecting the presence or absence of mutation of the codon 72 therein. The codon 72 adopts the sequence of CGC from the 5' side and forms arginine in the case of a wild type (hereinafter referred to also as "WT"), while the same mutates to the CCC sequence and forms proline in the case of a mutant (hereinafter referred to also as "MT"). The p53 gene is a gene frequently employed for cancer diagnosis, and it is said that many cancers are related to single nucleotide polymorphism of this gene. Therefore, the exon-4 gene was specifically amplified. The exon-4 gene which is the label object substance specifically aggregatively reacts with the gold nanoparticles modified with streptavidin.

The LAMP method known as an isothermal gene amplification method was employed for the amplification. The LAMP method is a low-priced and simple gene amplification reaction setting a plurality of types of primers with respect to a specific region of the target gene and causing a reaction at a constant temperature through a chain replacement reaction. This method is capable of gene amplification through one step by mixing the gene, the primers, an enzyme for synthesis of chain replacement type DNA and a substrate with each other and maintaining the mixture at a constant temperature around 65°C, has high amplification efficiency, and can amplify DNA to 10⁹ to 10¹⁰ times in 15 minutes to 1 hour. Further, the presence or absence of the target gene sequence can be rapidly and accurately determined by surveying the presence or absence of an amplification product, due to extremely high specificity.

In this Example, six types of primers, i.e., F3, B3, FIP, BIP, LoopF and LoopB were employed.
F3: 5'-CCCCGGACGATATTGAACAA-3' (sequence number 1)
B3: 5'-CCAGACGGAAACCGTAGCT-3' (sequence number 2)
FIP (WT): 5'-GCGGGGAGCAGCCTCGGTTCACTGAAGACCCAGG-3' (sequence number 3)
BIP (WT): 5'-CGCGTGGCCCCTGCGACAGGGGCCAGGAGG-3' (sequence number 4)
LoopF: 5'-Biotin-TGGCATTCTGGGAGCTTCA-3' (sequence number 5)
LoopB: 5'-Biotin-CGGCCCCTGCACCAG-3' (sequence number 6)
   As the primers in the mutant (MT), the following ones were employed for FIP and BIP:
FIP (MT): 5'-GGGGGGAGCAGCCTCGGTTCACTGAAGACCCAGG-3' (sequence number 7)
BIP (MT): 5'-CCCGTGGCCCCTGCGACAGGGGCCAGGAGG-3' (sequence number 8)

Human blood (WT) (1 µL) of a healthy person was employed as the sample. In advance of the amplification reaction, activation was performed in order to utilize whole blood. The liquid composition is as follows:
Buffer Solution: Ampdirect (by Shimadzu Biotech Japan) 25 µL
Sample (whole blood): 1 µL
The activation was performed at 95°C for 10 minutes, and a next reagent was mixed after the sample was cooled to ordinary temperature on standing, for preparing an amplification reaction solution. After the mixing step, this amplification reaction solution was introduced into a microfluidic circuit. This microfluidic circuit comprised specific reaction section 1, separation purification section 2 and detection section 3, as shown in Fig. 2(a).
   Enzyme: Bst DNA polymerase 0.25 µL
   Primers: (16 µM FIP, 16 µM BIP, 2 µM F3, 2 µM B3, 8 µM LoopF, 8 µM LoopB) 2.5 µL
   Distilled Water: 18.75 µL

### «Preliminary Examination»

It is known that the LAMP method is capable of reaction-specific amplification by design of primers, the target is hardly amplified when employing primers in MT including single nucleotide polymorphism and the target is selectively amplified only when employing primers in WT, this point was preliminarily examined. First, DNA extracted from the blood was used as the sample, and amplified with the following solution: In the preliminary examination, absorptions at a wavelength of 365 nm in LTV-visible spectra were observed with a Loopamp visual fluorescence detection reagent (by Eiken Chemical Co., Ltd.) with reference to reaction selectivity of primers in WT and MT. As to each of FIP and BIP, two types including the primers in WT and the primers in MT respectively were examined, and amplification reactions were performed at 63°C for 30 minutes.
Buffer Solution: Loopamp Reaction Buffer (by Eiken Chemical Co., Ltd.) 25 µL
Sample (extracted DNA): 1 µL
Enzyme: Bst DNA polymerase 0.25 µL
Primers: (16 µM FIP, 16 µM BIP, 2 µM F3, 2 µM B3, 8 µM LoopF, 8 µM LoopB) 2.5 µL
Loopamp Visual Fluorescence Detection Reagent 2.5 µL
Distilled Water: 16.25 µL

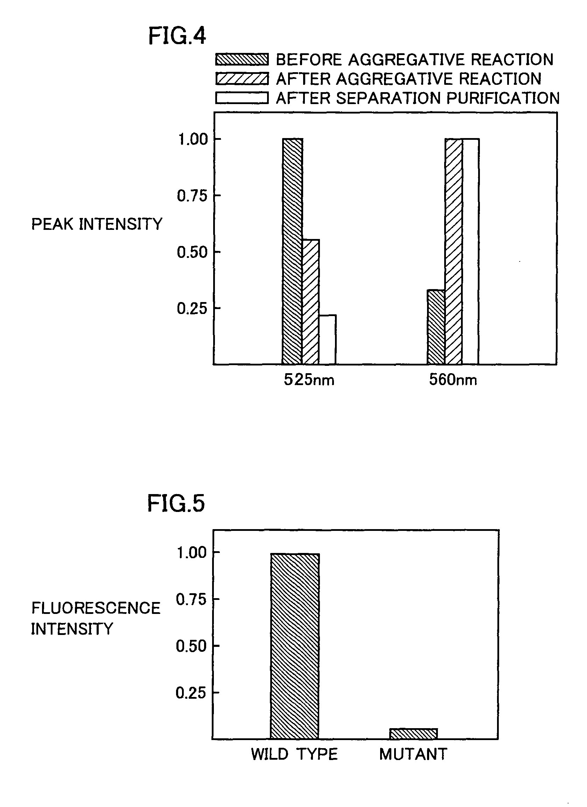

Fig. 5 shows fluorescence intensity levels of samples after gene amplification in the case of employing the primers in WT and the case of employing the primers in MT. The axis of ordinates in Fig. 5 shows relative values with reference to the fluorescence intensity in the case of employing the primers in WT. The p53 gene contained in the human blood of the healthy person is DNA of WT, and hence the amplification reaction selectively progresses in the case of employing the primers in WT, while no amplification takes place in the case of employing the primers in MT, and the fluorescence intensity ratio between the DNA of WT and the DNA of MT was at least 100 times, as shown in Fig. 5.

### <<Step of Forming Particulate Aggregate>>

On the basis of the results of the aforementioned preliminary examination, the following steps were specifically carried out in this Example:

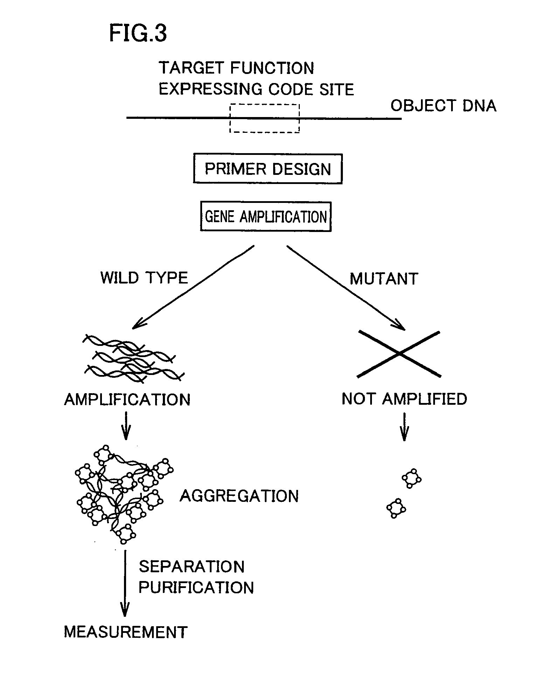

Fig. 3 shows the outline of the specific steps in this Example. As shown in Fig. 3, each tested DNA was prepared from the p53 gene, the codon 72 was selected as the target function expressing code site, the primers were thereafter designed, and the exon-4 gene in the p53 gene was amplified by the LAMP method under the aforementioned conditions.

In this Example, two types of the aforementioned amplification reaction solutions containing WT and MT respectively were adjusted as to FIP and BIP. The gene amplification reactions were performed at a constant temperature of 63°C for 30 minutes. The amplified DNA in the two types of amplification reaction solutions after the gene amplification reactions was biotinized by amplification of oligos with previously biotin-modified primers.

The reactions between the two types of amplification reaction solutions containing the biotinized DNA and gold nanoparticles modified with streptavidin were examined. A DNA-gold nanoparticle mixed solution was prepared by mixing 10 µL of each of the biotinized amplification reaction solutions into 10 µL of an aqueous solution of the aforementioned gold nanoparticles, and the degree of aggregation after performing a reaction at ordinary temperature for 10 minutes was visually confirmed. The DNA-gold nanoparticle mixed solution amplified from the primers in WT changed the color from red to purple, and it was confirmed that the aggregation reaction was in progress. In the DNA-gold nanoparticle mixed solution amplified from the primers in MT, on the other hand, no color change was recognized before and after the addition of the gold nanoparticles. The DNA-gold nanoparticle mixed solution amplified from the primers in WT exhibiting the aggregative reaction was centrifugally separation-purified, and an aggregated sample was recovered. After the recovery of the aggregated sample, a low salt concentration buffer solution was introduced from another chamber in the microfluidic circuit, and the gold nanoparticle aggregate was re-diffused in the buffer solution.

### <<Step of Separation-Purifying Particulate Aggregate>>

Centrifugal separation purification as to the aforementioned aggregated sample was performed with a commercially available general centrifugal separator, while setting the condition to 3000 rpm to 30000 rpm.

### <<Detection/Measurement>>

The gold nanoparticle aggregate was detected. The solution containing the gold nanoparticle aggregate prepared with the primers in WT was introduced into an electrochemical reaction chamber having an electrode of ITO. The surface of the electrode of ITO had been previously modified with 3-aminopropyl triethoxysilane (APS), for forming an amino terminal monolayer film. The amino terminal monolayer film was formed by dipping a substrate in an APS solution of 1 percent by mass for 10 minutes. The gold nanoparticle aggregate was fixed to the substrate surface due to interaction between the amino terminal monolayer film formed on the electrode of ITO and biotin-streptavidin present in the gold nanoparticle aggregate. When cyclic voltammetric measurement was performed after this fixation, a reduction peak derived from gold was observed from the cyclic voltammogram. In the case of employing the primers in MT, on the other hand, a peak derived from the gold nanoparticle aggregate was hardly observed on the surface of the electrode of ITO, and a significant difference was recognized.

Measurement with a surface plasmon resonance apparatus was performed. Fig. 4 shows results obtained by measuring the gold nanoparticle aggregate prepared with the primers in WT fixed to the surface of the electrode of ITO by the aforementioned method. The peak intensity values on the axis of ordinates in Fig. 4 are relative values with reference to each peak intensity of absorptions at wavelengths 525 nm and 560 nm obtained by the stock solution (concentration: 0.16 %) of the gold nanoparticles with the surface plasmon resonance apparatus. Specific plasmon absorption peaks resulting from isolated states of the gold nanoparticles appear around the wavelength of 525 nm, while plasmon absorption peaks specific to gold are observed around the wavelength of 560 nm. As to each wavelength, absorption peaks before the aggregative reaction, after the aggregative reaction and after the separation purification were classified in Fig. 4. As shown in Fig. 4, the plasmon absorption peaks specific to gold were observed and the peak intensity levels increased to 3 to 4 times by the aggregative reaction in the case of employing the primers in WT. As to the absorption peaks at the wavelength of 525 nm, the peak intensity levels gradually reduced following the aggregation and the separation purification. In the solution containing the gold nanoparticle aggregate prepared with the primers in MT, on the other hand, peaks derived from gold were hardly observed. According to the aforementioned electrochemical measurement and surface plasmon resonance measurement, the gold nanoparticles were detectable in an unlabeled manner, and it was possible to separation-purify the particulate aggregate by the selective amplification reaction of the wild type and the mutant.

### (Example 2)

In this Example, pepsinogen I which is one of cancer tumor markers was detected by an antigen-antibody reaction employing gold nanoparticles. Human serum was employed as a specimen, and gold nanoparticles of 20 nm in average particle diameter modified with a mouse monoclonal pepsinogen I antibody were employed as particulates specifically reacting with pepsinogen I in the specimen. When pepsinogen I was present in the specimen, the gold nanoparticles modified with the mouse monoclonal pepsinogen I antibody and pepsinogen I specifically reacted with each other and formed a particulate aggregate accompanied by a sandwich reaction. The color of the solution containing the particulate aggregate changed from red to purple due to the formation of the particulate aggregate. Thereafter the formed particulate aggregate was centrifugally separation-defined. The centrifugal separation purification was performed in an apparatus similar to that in Example 1 under a similar condition.

As a result of performing similar treatment while changing the concentration of pepsinogen I in the specimen by 0 nm/mL to 200 nm/mL, the color of the solution containing the particulate aggregate after the aggregative reaction gradually changed from red to purple following the increase of the concentration of pepsinogen I, and as a result of measuring the absorption spectrum of this solution with an ultraviolet visible spectrometer, the absorption wavelength peak changed from initial 525 nm to 560 nm to 620 nm after the aggregative reaction. Therefore, it was possible to quantitatively confirm that the quantity of the formed particulate aggregate varied with the quantity of pepsinogen I in the specimen, and it was recognized that the label object substance can be not only qualitatively but also quantitatively analyzed by the method according to the present invention.

Considering along with the results of Examples, cancer-derived gene mutation and a cancer tumor marker can be detected on the same chip having a similar pattern, and it has been recognized that the method and the microfluidic circuit according to the present invention are effective for ulterior advanced medical diagnosis and early medical care.

The embodiment and Examples disclosed this time are to be considered as illustrative in all points and not restrictive. The range of the present invention is shown not by the above description but by the scope of claims for patent, and it is intended that all modifications within the meaning and range equivalent to the scope of claims for patent are included.

### INDUSTRIAL APPLICABILITY

A label object substance in biomolecules or a bio-associated substance can be simply qualitatively or quantitatively tested, to be useful for ulterior advanced medical care and early medical care of a gene disease.

## Claims

1. A separation purification method utilizing an aggregative reaction of particulates, comprising the steps of:
forming a particulate aggregate by a reaction between particulates modified with a labeling substance specifically reacting with a label object substance and biomolecules or a bio-associated substance containing the label object substance; and
separation-purifying said particulate aggregate.

2. The separation purification method according to claim 1, wherein
said particulates are particles of gold having an average particle diameter of 1 nm to 500 nm.

3. The separation purification method according to claim 1, centrifugally separation-purifying said particulate aggregate.

4. The separation purification method according to claim 1, wherein
said label object substance contains an amplified gene prepared by amplifying a wild type gene or a mutant gene contained in body fluid or a tissue segment.

5. The separation purification method according to claim 4, wherein
said body fluid or said tissue segment contains the label object substance.

6. A microfluidic circuit comprising a specific reaction section (1), a separation purification section (2) and a detection section (3), wherein
said specific reaction section (1) forms a particulate aggregate by a specific reaction between particulates modified with a labeling substance specifically reacting with a label object substance and biomolecules or a bio-associated substance containing the label object substance,
said separation purification section (2) separation-purifies the formed particulate aggregate, and
said detection section (3) detects the separation-purified particulate aggregate by a nonfluorescence method.

7. The microfluidic circuit according to claim 6, employed for detecting gene mutation including single nucleotide polymorphism.

8. The microfluidic circuit according to claim 6, employed for qualitatively or quantitatively detecting a cancer tumor marker.

9. The microfluidic circuit according to claim 6, wherein
at least a part of the specific reaction section (1), the separation purification section (2) and the detection section (3) includes a plurality of chambers.

10. The microfluidic circuit according to claim 6, centrifugally feeding fluid between the specific reaction section (1), the separation purification section (2) and the detection section (3).

11. The microfluidic circuit according to claim 6, wherein
said detection section (3) has a detection surface for fixing the particulate aggregate.

12. The microfluidic circuit according to claim 6, wherein
said particulates contain particles of gold and particles of noble metal other than gold.

13. The microfluidic circuit according to claim 6, wherein
said detection section (3) has an electrode for an electrochemical reaction.

14. The microfluidic circuit according to claim 6, wherein
said detection section (3) performs detection by surface plasmon resonance.

15. The microfluidic circuit according to claim 6, quantitatively or qualitatively
detecting one or both of a cancer-associated gene and a tumor marker in the same chip.
